# EUROPEAN PATENT SPECIFICATION

(11) **EP 3 757 025 B1**
(45) Date of publication and mention of the grant of the patent: **15.02.2023**
(21) Application number: 20178827.0
(22) Date of filing: 08.06.2020
(51) Int. Cl.: B65B 55/02, B67C 7/00

(54) **A PLANT FOR TREATING RECEPTACLES ADAPTED TO CONTAIN A POURABLE PRODUCT**
ANLAGE ZUM BEHANDELN VON BEHÄLTERN, DIE DAFÜR AUSGELEGT SIND, EIN FLIESSFÄHIGES PRODUKT ZU ENTHALTEN
INSTALLATION DE TRAITEMENT DE RÉCIPIENTS CONÇUS POUR CONTENIR UN PRODUIT VERSABLE

(30) Priority: 26.06.2019 IT 201900010134
(43) Date of publication of application: 30.12.2020
(73) Proprietor: Sidel Participations, 76930 Octeville-sur-Mer (FR)
(72) Inventor: LICHNEWSKY, Lionel, 43126 Parma (IT); FUSARO, Alessandro, 43126 Parma (IT)
(74) Representative: Sidel Group

(56) References cited:
- EP-A1- 1 790 571
- EP-A1- 2 246 176
- EP-A1- 3 088 313
- EP-A1- 3 428 077
- WO-A1-98/43878
- WO-A1-2018/092441
- DE-A1-102016 213 421
- US-A1- 2008 210 000

## Description

### TECHNICAL FIELD

The present invention relates to a plant for treating receptacles, for example bottles made of plastic or the like, adapted to be filled with a pourable product, preferably a pourable food product, even more preferably sterilised.

In particular, the present invention relates to a plant for producing the above-mentioned receptacles from known preforms, for filling the receptacles with the pourable product and for applying caps to the receptacles (i.e., capping them).

### BACKGROUND ART

Plants for treating receptacles are known, which are adapted to contain, in particular to be filled with, a pourable product, preferably a pourable food product, even more preferably sterilised.

Generally, receptacles treated in the above-mentioned plants are defined by bottles, containers or the like.

In the present description, by the term "treated", "treatment" or the like, it is to be understood, either alone or in combination, the forming, filling and capping processes of the above-mentioned receptacles.

Typically, a treatment plant of the above-mentioned type essentially comprises:
- a forming unit for forming receptacles from known preforms, usually made of plastic material, each of which is apt to be shaped to obtain respective receptacles;
- a filling unit receiving in input the formed receptacles, preferably sterilised, from the forming unit and configured to fill said receptacles with the pourable product; and
- a capping unit receiving in input receptacles filled by the filling unit and configured to apply a cap to each individual receptacle and to feed the receptacles, thus filled and closed, to a conveyor device for the possible subsequent labelling, packaging and storage operations.

The need for ensuring a suitable aseptic condition of the receptacles during the treatment process is known in the art, in order to guarantee quality and safety standards envisaged for the consumers.

Consequently, plants of the above-mentioned type further comprise an isolation chamber, housing at least partially, at least the filling unit and the capping unit and configured to internally delimit a controlled atmosphere, so that the filling and capping units can operate in sterile and/or aseptic conditions.

The isolation chamber therefore defines a sterile and/or aseptic environment of the treatment plant, required to guarantee the above-mentioned operating conditions.

In order to create said sterile and/or aseptic conditions, a typical treatment plant further comprises a plurality of filtering units, each of which is configured to filter the air to be introduced into the isolation chamber, usually sucked-up from the external environment by suction means, such as for example fans, creating, in this manner, the controlled atmosphere inside the isolation chamber.

As is known, the filtering units are operatively interposed between the external environment and the isolation chamber, in particular arranged at a boundary wall of the isolation chamber that separates the external environment from the controlled environment.

In particular, each filtering unit may comprise one or more filters of a known type (for example, HEPA or ULPA filters) and one or more fans operatively coupled to the corresponding filters. The fans convey, in use, the air from the external environment towards the relative filters; said air is then filtered (sterilised and/or made aseptic) and introduced inside the isolation chamber.

The isolation chamber is configured, therefore, to operate in overpressure conditions with respect to the external environment.

In some cases, also the above-mentioned forming unit is housed in the same isolation chamber or in a respective further isolation chamber of the above-mentioned type, the latter typically being fed with filtered air but not containing a sterile and/or aseptic controlled atmosphere.

The need to periodically control the above-mentioned filtering unit is known in the art, in order to guarantee the aforesaid quality and safety standards (aseptic standards usually required by the competent authorities of the sector).

It is therefore necessary to perform periodic controls, typically performed manually by an operator, which entails stoppage of the treatment plant, emptying the isolation chamber from the relative production batch, opening the isolation chamber, with corresponding interruption of the aseptic/sterile conditions, and the required transient period after controls for recalibrating the optimal operating conditions in order to resume the treatment process.

WO9843878 discloses a carton filling machine comprising a compartmentalized clean air system for providing a downward unidirectional flow of clean air into a chamber that substantially encloses one of the machine stations.

Albeit the plants of the above-mentioned type represent a valid solution for treating - forming, filling and capping - receptacles adapted to contain a pourable product, the Applicant has observed that these are still susceptible to further improvements. In particular, the need is felt to reduce the stoppages of the plant, avoid recalibration times and improve the aseptic quality inside the isolation chamber.

### OBJECT AND SUMMARY OF THE INVENTION

The object of the present invention is to achieve a plant for treating receptacles, which is more reliable and less costly, and enables to meet at least some of the needs specified above and connected to the treatment plants of the known type.

According to the invention, this object is achieved by a treatment plant as claimed in the attached claims.

### BRIEF DESCRIPTION OF THE DRAWINGS

For a better understanding of the present invention, a preferred non-limiting embodiment thereof will be described in the following, purely by way of example and with the aid of the attached drawings, wherein;

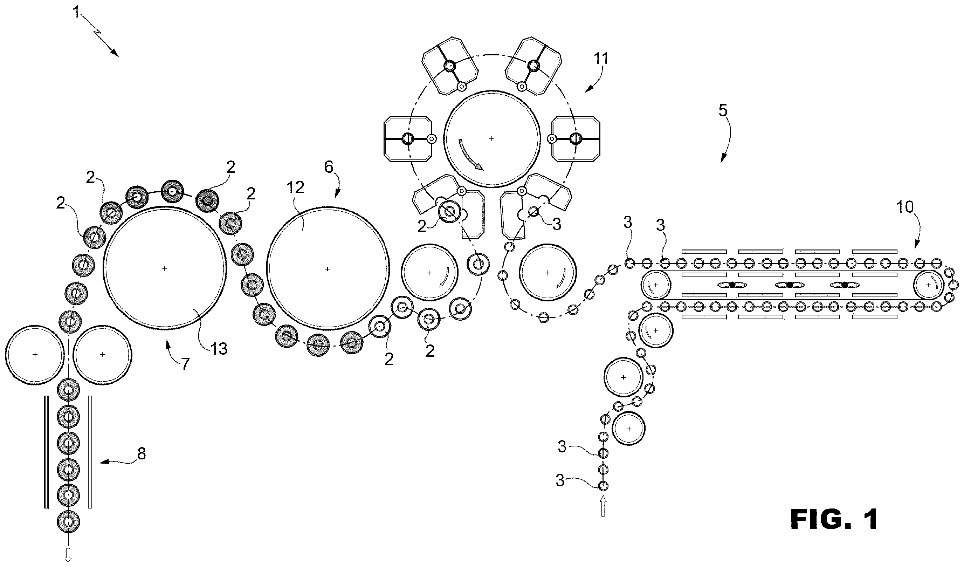

- figure 1 is a schematic top plan view, with parts removed for clarity, of a treatment plant achieved according to the present invention; and
- figure 2 is a larger-scale, schematic side view, with parts removed for clarity, of the treatment plant of figure 1.

### BEST MODE FOR CARRYING OUT THE INVENTION

With reference to the attached figures, number 1 indicates as a whole a plant for treating receptacles 2 which are adapted to contain a pourable product, preferably a pourable food product, even more preferably sterilised, such as carbonated beverages (for example, sparkling water, soft drinks and beer) or still beverages (for example water, fruit juices, tea, sport drinks, wine, emulsions, yoghurt, etc..)

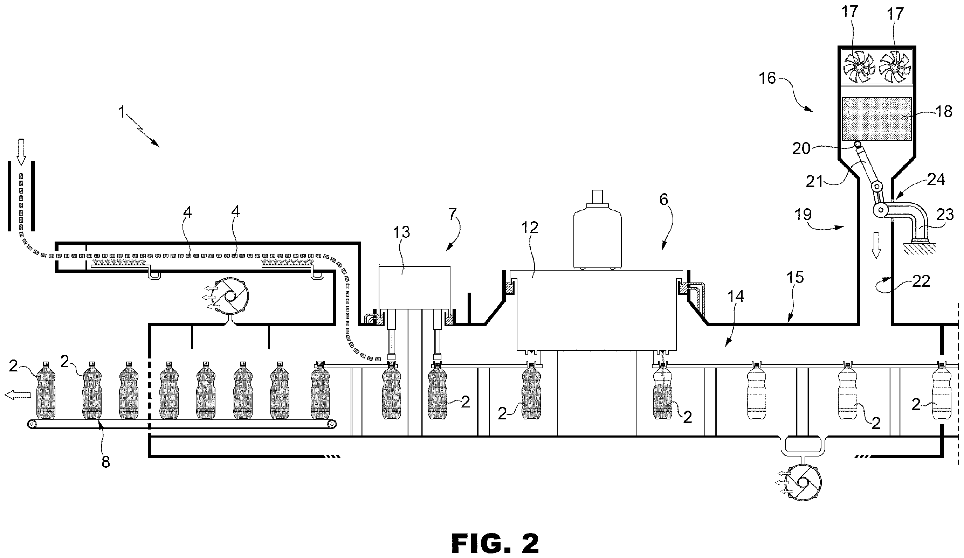

In particular, the plant 1 is adapted to produce receptacles 2, in the example described bottles made of plastic, from preforms 3, to fill receptacles 2 with the pourable product and to subsequently cap the filled receptacles with the respective caps 4 (figure 2).

In this regard, the plant 1 comprises, at least:
- a forming unit 5, in particular a blower, configured to produce receptacles 2 from the preforms 3 by means of a blow-forming/compression moulding process, known per se and not described in detail;
- a filling unit 6 that receives, in use, the receptacles 2 formed by the forming unit 5, preferably at least internally sterilised, and is configured to fill, in a known manner, said receptacles 2 with the pourable product; and
- a capping unit 7 that receives, in use, the receptacles 2 filled by the filling unit 6 and is configured to apply a cap 4 to each individual receptacle 2 and to feed said filled and closed receptacles 2 to an outlet conveyor 8.

In an alternative embodiment not illustrated, the plant 1 comprises only one or any combination of the forming unit 5, filling unit 6 and capping unit 7.

Therefore, in the following description, by the term "treatment" it is intended the forming and/or filling and/or capping process of the receptacles 2.

As is known, the forming unit 5 comprises a kiln 10 for preheating the preforms 3 and a forming device 11, arranged operatively downstream of the kiln 10 and configured to shape the previously heated preforms 3, so as to obtain the receptacles 2 of the desired shape.

Preferably, forming takes place by means of blowing inside the preheated preforms 3, which expand until assuming the pre-established shape imparted thereto by the forming device 11.

The filling unit 6 is arranged operatively downstream of the forming unit 5 and comprises a conveyor, in the example described a carousel 12, configured to receive the formed receptacles 2, to feed them along a transfer path, shaped as an arc of a circle and to fill them with the pourable product, in a manner known and not described in detail.

The capping unit 7 is arranged operatively downstream of the filling unit 6 and comprises a conveyor, in the example described a carousel 13 configured to receive the caps 4 from an inlet conveyor not described in detail, to receive the receptacles 2 filled by the carousel 12, to feed them along a corresponding transfer path shaped as an arc of a circle, to support them in a rotatable manner and cap them with the corresponding caps 4, in a manner known and not described in detail.

After being capped, the receptacles 2 are then fed to the outlet conveyor 8 for possible subsequent labelling, packaging and storage operations.

In order to guarantee that the treatment process is carried out according to the pre-established foodstuff quality and safety standards, the plant 1 further comprises an isolation chamber 14 (figure 2) housing, at least partially and according to this preferred and non-limiting embodiment, the filling unit 6 and the capping unit 7.

In use, the isolation chamber 14 is configured to delimit, i.e. to contain therein, a controlled atmosphere controlled in sterile and/or aseptic conditions. Therefore, the isolation chamber 14 defines a sterile and/or aseptic environment of the plant 1. In this manner, the filling unit 6 and the capping unit 7 can operate in sterile and/or aseptic conditions, to guarantee the aforementioned standards.

Conveniently, the isolation chamber 14 is delimited by a boundary wall 15 that separates and seals in a fluid tight manner the environment inside the isolation chamber 14 from the external environment.

In order to create the above-mentioned sterile and/or aseptic conditions inside the isolation chamber 14 and, thus, constitute the controlled atmosphere, the treatment plant 1 comprises at least a filtering unit 16 adapted to filter a gas, in this specific example air, to be introduced inside the isolation chamber 14.

In detail, as can be seen in figure 2, the filtering unit 16 comprises:
- a filtering member 18, for example a HEPA or ULPA filter, configured to filter air coming from the external environment, sterilise it/make it aseptic and introduce it to the inside of the isolation chamber 14; and
- an air convection member, preferably one or more fans 17, each apt to convey the air to be filtered from the external environment towards the filtering member 18.

In use, the air, after having been conveyed by the fan 17 towards the filtering member 18, is filtered/sterilised and subsequently introduced inside the isolation chamber 14.

As the isolation chamber 14 is sealed, it is configured to operate in overpressure conditions with respect to the external environment.

Conveniently, the filtering unit 16 is arranged at the wall 15, in particular is fixed to the wall 15, with the fan 17 facing the external environment and the filtering member 18 facing the environment inside the isolation chamber 14.

In greater detail, the treatment plant 1 comprises a duct 19 that fluidically connects the filtering unit 16 with the isolation chamber 14.

More precisely, the duct 19 is adapted to contain, in use, the controlled atmosphere and, therefore, can be considered an extension (and therefore an integral part) of the isolation chamber 14. Consequently, the wall 15 externally delimits the duct 19.

In an alternative embodiment not illustrated, the treatment plant 1 is not provided with any duct 19 and the filtering unit 16 is arranged directly on the wall 15 delimiting the isolation chamber 14.

According to the invention, the treatment plant 1 further comprises a test device 20 housed inside the isolation chamber 14, immersed, in use, in the controlled atmosphere and configured to perform one or more test operations on the filtering unit 16, in particular on the filtering member 18.

In detail, the test (or check and/or control) operations comprise:
- the detection, by means of the test device 20, of particles of contaminating agents possibly present in the controlled atmosphere, and therefore in the isolation chamber 14, which could compromise the sterile and/or aseptic conditions, and/or the measurement of an amount thereof, for example parts by million (ppm); and/or
- the positioning of an initially sterile culture medium (known per se are not illustrated) inside the isolation chamber 14, the medium being configured to support and house one or more microbiological cultures, and the analysis of the culture medium after a certain pre-set period of time of immersion in the controlled atmosphere.

Preferably, the test device 20 comprises a particle counter.

It is specified that by the expression "culture medium" it is intended, in the present description, any element adapted to support and/or host one or more known microbiological cultures.

In an embodiment, said culture medium could be defined by one or more strips of support material that can be contaminated with a known microbiological culture.

In an embodiment, the test device 20 comprises means for depositing the above-mentioned culture medium, defined for example by one (or more) of the above-mentioned, preferably adhesive, strips, and means for analysing the culture medium.

In practice, the initially sterile strip, is adapted to be arranged inside the duct 19, and therefore in the isolation chamber 14, at the filtering unit 16. Preferably, the test device 20 is configured to deposit the strip downstream of a sterilising unit of the filtering unit 16, arranged upstream of the filtering member 18 and configured to sterilise the filtering member 18.

After a certain period of time, the test device 20 is configured to analyse the strip and to detect (check) the presence or absence of possible microbiological cultures formed, during the treatment process, on the strip itself.

In this manner, the efficiency of the sterilising unit, and therefore of the filtering unit 16, can be easily checked during the treatment process and without interrupting the treatment process itself.

In light of what has been described above, the test device 20 is configured to carry out:
- quality controls of the controlled atmosphere contained, in use, inside the isolation chamber 14 and, therefore, of the correct operation of the filtering unit 16; and/or
- a test to check the efficiency of the filtering unit 16 during the treatment process.

According to another aspect of the present invention, the test device 20 is movable inside the isolation chamber 14 and, therefore, inside the controlled atmosphere contained, in use, therein.

In detail, the test device 20 is carried by a robotic arm 21, conveniently arranged inside the isolation chamber 14 and thus apt to be immersed inside the controlled atmosphere.

Preferably, the robotic arm 21 is arranged in the duct 19 and is appropriately positioned so as to control the movement of the test device 20 at the filtering member 18 of the filtering unit 16.

In use, the robotic arm 21 is controllable, for example by means of a control unit, known and not illustrated, to move the test device 20 at, i.e. in the proximity of, the filtering member 18 to detect and measure a quantity of the particles of contaminating agents possibly present downstream of the filtering element 18 and that, therefore, are introduced in the duct 19 and in the isolation chamber 14.

The above-mentioned detection can be carried out during the treatment process (filling and/or capping) of receptacles 2, without the need to stop the treatment plant 1.

Therefore, the above-mentioned test operations are carried out automatically by means of the robotic arm 21 and without the manual intervention of any operator, during the filling and capping processes.

According to this preferred and non-limiting embodiment, the robotic arm 21 is fixed to an internal wall 22 of the duct 19 in particular is flanged to the internal wall 22, and is coupled in a mobile manner to a fixed support base 23 arranged outside of the duct 19 and therefore outside of the isolation chamber 14.

Expediently, the robotic arm 21 is coupled to the support base 23 by means of a coupling flange 24, schematically illustrated in figure 2 and comprising a fluid sealing system adapted to seal the coupling area and configured to enable the movement of the robotic arm 21 with respect to the support base 23 thus guaranteeing the fluid tight seal between the flange 24 and the wall 22, i.e. between the environment inside the duct 19, and therefore in the isolation chamber 14, and the external environment.

Conveniently, the test device 20 is mounted on a free end portion of the robotic arm 21 so as to be able to easily reach the pre-set operating area.

Expediently, the robotic arm 21 is provided with six degrees of freedom.

From an examination of the features of the treatment plant 1 according to the present invention the advantages that it allows to obtain are evident.

In particular, thanks to the presence of the test device 20 arranged in a mobile manner inside the isolation chamber 14 and carried by the robotic arm 21, which is also arranged inside the isolation chamber 14, it is possible to carry out the test operations during, and without interrupting, the treatment process of the receptacles 2 (and therefore on the fly), in an automatic manner. In this manner, the stoppages of the treatment plant 1 are drastically reduced, and the transitory times required for recalibration of the optimal sterile and/or aseptic operating conditions of the controlled atmosphere, are avoided.

Furthermore, this results in an improved aseptic and/or sterile condition of the controlled atmosphere, since there is no interruption of the aseptic and/or sterile conditions each time it is necessary to carry out the test operations.

In addition, thanks to the configuration described above, tests can be carried out on the efficiency of the filtering unit 16 during, and without interrupting, the treatment process, which otherwise, in the absence of the configuration according to the present invention, would require the presence of an appropriate separation structure. The architecture of the treatment plant 1 is, therefore, extremely simplified.

It is clear that modifications and variations can be made to the treatment plant 1 described and illustrated here without thereby departing from the scope defined by the claims.

In particular, the treatment plant 1 could comprise, furthermore, a further isolation chamber that houses the forming unit 5 and is configured to contain a respective controlled atmosphere.

In this case, the controlled atmosphere of said isolation chamber is preferably formed by filtered air but not sterilised and/or aseptic air.

In order to create said controlled atmosphere, the treatment plant 1 could comprise a further filtering unit for filtering the air to be introduced inside the isolation chamber.

Conveniently, the treatment 1 could comprise a further robotic arm, substantially identical to the robotic arm 21 and carrying a test device 20, to carry out test operations on the further filtering unit.

In addition, the treatment plant 1 could comprise a single isolation chamber housing the forming 5, filling 6 and capping 7 units and supplied by a single filtering unit.

Furthermore, the treatment plant 1 could comprise two or more robotic arms 21 carrying as many test devices 20 and arranged inside the isolation chamber 14.

## Claims

1. A plant (1) for treating receptacles (2) adapted to contain a pourable product comprising:
- at least a treatment unit (5, 6, 7) apt to carry out a treatment process on said receptacles (2);
- an isolation chamber (14) housing said treatment unit (5, 6, 7) and containing, in use, a controlled atmosphere controlled in sterile and/or aseptic conditions; and
- a filtering unit (16) configured to filter a gas to be introduced into said isolation chamber (14) for constituting said controlled atmosphere;
wherein said treatment plant (1) further comprises a test device (20) housed inside said isolation chamber (14), immersed, in use, in said controlled atmosphere and configured to perform one or more test operations on said filtering unit (16);
wherein the test operations comprise:
- the detection, by means of the test device (20), of particles of contaminating agents possibly present in the controlled atmosphere, and therefore in the isolation chamber (14), which could compromise the sterile and/or aseptic conditions, and/or the measurement of an amount thereof, for example parts by million (ppm); and/or
- the positioning of an initially sterile culture medium inside the isolation chamber (14), the medium being configured to support and house one or more microbiological cultures, and the analysis of the culture medium after a certain pre-set period of time of immersion in the controlled atmosphere;
wherein said test device (20) is movable within said isolation chamber (14);
**characterized in that** said test device (20) is carried by a robotic arm (21).

2. The treatment plant as claimed in claim 1, wherein said test device (20) is movable within said isolation chamber (14) at said filtering unit (16).

3. The treatment plant as claimed in any of the previous claims, wherein said robotic arm (21) is arranged inside said isolation chamber (14).

4. The treatment plant as claimed in any of the previous claims, further comprising a duct (19) fluidically connecting said filtering unit (16) to said isolation chamber (14) and apt to contain, in use, said controlled atmosphere; wherein said robotic arm (21) is arranged in said duct (19).

5. The treatment plant as claimed in any one of the preceding claims, wherein said test operations comprise the detection, by means of said test device (20), of particles of contaminating agents in said controlled atmosphere and/or the measurement of a quantity of the latter.

6. The treatment plant as claimed in any one of the preceding claims, wherein said test operations comprise the positioning of a culture medium carrying one or more microbiological cultures inside said isolation chamber (14) and the analysis of said culture medium after having been immersed in the controlled atmosphere for a certain period of time.

7. The treatment plant as claimed in any one of the preceding claims, wherein said treatment unit (5, 6, 7) is defined by:
- a forming unit (5) for forming said receptacles (2) from preforms (3); or
- a filling unit (6) for filling said receptacles (2) with a pourable product; or
- a capping unit (7) for capping said receptacles (2) containing said pourable product; or
- a combination thereof.

## Patentansprüche

1. Anlage (1) zum Behandeln von Behältern (2), die dafür ausgelegt sind, ein fließfähiges Produkt zu enthalten, umfassend:
- mindestens eine Behandlungseinheit (5, 6, 7), die geeignet ist, um einen Behandlungsprozess an dem Behälter (2) durchzuführen;
- eine Isolierkammer (14), in der die Behandlungseinheit (5, 6, 7) untergebracht ist, und die bei Gebrauch eine kontrollierte Atmosphäre enthält, die so kontrolliert wird, dass sterile und/oder aseptische Bedingungen vorliegen; und
- eine Filtrationseinheit (16), die ausgestaltet ist, um ein in die Isolierkammer (14) einzubringendes Gas zu filtrieren, welches die kontrollierte Atmosphäre bilden soll;
wobei die Behandlungsanlage (1) des Weiteren eine Testvorrichtung (20) umfasst, die innerhalb der Isolierkammer (14) untergebracht ist, bei Gebrauch in die kontrollierte Atmosphäre eintaucht und ausgestaltet ist, um an der Filtrationseinheit (16) einen oder mehrere Testvorgänge durchzuführen; wobei die Testvorgänge umfassen:
- die Detektierung von Partikeln aus Kontaminationsmitteln, die möglicherweise in der kontrollierten Atmosphäre und dadurch in der Isolierkammer (14) vorhanden sind, mittels der Testvorrichtung (20), wodurch die sterilen und/oder aseptischen Bedingungen gefährdet sein könnten, und/oder die Messung einer Menge davon, beispielsweise Teile pro Million (ppm); und/oder
- die Positionierung eines anfangs sterilen Kulturmediums innerhalb der Isolierkammer (14), wobei das Medium ausgestaltet ist, um eine oder mehrere mikrobielle Kulturen zu unterstützen und unterzubringen, und die Analyse des Kulturmediums nach einer vorgewählten Zeitperiode des Eintauchens in die kontrollierte Atmosphäre;
wobei die Testvorrichtung (20) innerhalb der Isolierkammer (14) beweglich ist;
**dadurch gekennzeichnet, dass** die Testvorrichtung (20) von einem Roboterarm (21) getragen wird.

2. Behandlungsanlage nach Anspruch 1, wobei die Testvorrichtung (20) innerhalb der Isolierkammer (14) an der Filtrationseinheit (16) beweglich ist.

3. Behandlungsanlage nach einem der vorhergehenden Ansprüche, wobei der Roboterarm (21) innerhalb der Isolierkammer (14) angeordnet ist.

4. Behandlungsanlage nach einem der vorhergehenden Ansprüche, des Weiteren umfassend einen Schacht (19), der die Filtrationseinheit (16) fließtechnisch mit der Isolierkammer (14) verbindet und geeignet ist, um bei Gebrauch die kontrollierte Atmosphäre zu enthalten; wobei der Roboterarm (21) in dem Schacht (19) angeordnet ist.

5. Behandlungsanlage nach einem der vorhergehenden Ansprüche, wobei die Testvorgänge die Detektierung von Partikeln von Kontaminationsmitteln in der kontrollierten Atmosphäre und/oder die Messung einer Menge von letzteren durch die Testvorrichtung (20) umfassen.

6. Behandlungsanlage nach einem der vorhergehenden Ansprüche, wobei die Testvorgänge die Positionierung eines Kulturmediums zum Tragen von einer oder mehrerer mikrobiologischer Kulturen innerhalb der Isolierkammer (14) und die Analyse des Kulturmediums umfassen, nachdem es für eine bestimmte Zeitperiode in die kontrollierte Atmosphäre eingetaucht worden ist.

7. Behandlungsanlage nach einem der vorhergehenden Ansprüche, wobei die Behandlungseinheit (5, 6, 7) definiert ist durch:
- eine Formungseinheit (5) zum Formen der Behälter (2) aus Vorformlingen (3); oder
- eine Fülleinheit (6) zum Füllen der Behälter (2) mit einem gießbaren Produkt; oder
- eine Schließeinheit (7) zum Schließen der Behälter (2), die das gießbare Produkt enthalten; oder
- eine Kombination davon.

## Revendications

1. Installation (1) pour le traitement de réservoirs (2) conçus pour contenir un produit pouvant être versé comprenant :
- au moins une unité de traitement (5, 6, 7) pouvant effectuer un processus de traitement sur lesdits réservoirs (2) ;
- une chambre d'isolement (14) logeant ladite unité de traitement (5, 6, 7) et contenant, lors de l'utilisation, une atmosphère contrôlée dans des conditions stériles et/ou aseptiques ; et
- une unité de filtration (16) conçue pour filtrer un gaz à introduire dans ladite chambre d'isolement (14) pour constituer ladite atmosphère contrôlée ;
dans laquelle ladite installation de traitement (1) comprend en outre un dispositif de test (20) logé à l'intérieur de ladite chambre d'isolement (14), immergé, lors de l'utilisation, dans ladite atmosphère contrôlée et conçu pour effectuer une ou plusieurs opérations de test sur ladite unité de filtration (16) ;
dans laquelle les opérations de test comprennent :
- la détection, au moyen du dispositif de test (20), de particules d'agents contaminants éventuellement présentes dans l'atmosphère contrôlée, et de ce fait dans la chambre d'isolement (14), qui pourraient compromettre les conditions stériles et/ou aseptiques, et/ou la mesure d'une quantité de celles-ci, par exemple en parties par million (ppm) ; et/ou
- le positionnement d'un milieu de culture initialement stérile à l'intérieur de la chambre d'isolement (14), le milieu étant conçu pour supporter et loger une ou plusieurs cultures microbiologiques, et l'analyse du milieu de culture après une certaine durée prédéfinie d'immersion dans l'atmosphère contrôlée ;
dans laquelle ledit dispositif de test (20) est mobile à l'intérieur de ladite chambre d'isolement (14) ;
**caractérisée en ce que** ledit dispositif de test (20) est porté par un bras robotisé (21).

2. Installation de traitement selon la revendication 1, dans laquelle
ledit dispositif de test (20) est mobile à l'intérieur de ladite chambre d'isolement (14) au niveau de ladite unité de filtration (16).

3. Installation de traitement selon l'une quelconque des revendications précédentes, dans laquelle ledit bras robotisé (21) est agencé à l'intérieur de ladite chambre d'isolement (14).

4. Installation de traitement selon l'une quelconque des revendications précédentes, comprenant en outre un conduit (19) reliant fluidiquement ladite unité de filtration (16) à ladite chambre d'isolement (14) et pouvant contenir, lors de l'utilisation, ladite atmosphère contrôlée ;
dans laquelle ledit bras robotisé (21) est agencé dans ledit conduit (19).

5. Installation de traitement selon l'une quelconque des revendications précédentes, dans laquelle lesdites opérations de test comprennent la détection, au moyen dudit dispositif de test (20), de particules d'agents contaminants dans ladite atmosphère contrôlée et/ou la mesure d'une quantité de ces dernières.

6. Installation de traitement selon l'une quelconque des revendications précédentes, dans laquelle lesdites opérations de test comprennent le positionnement d'un milieu de culture portant une ou plusieurs cultures microbiologiques à l'intérieur de ladite chambre d'isolement (14) et l'analyse dudit milieu de culture après avoir été immergé dans l'atmosphère contrôlée pendant une certaine durée.

7. Installation de traitement selon l'une quelconque des revendications précédentes, dans laquelle ladite unité de traitement (5, 6, 7) est définie par :
- une unité de mise en forme (5) pour former lesdits réservoirs (2) à partir de préformes (3) ; ou
- une unité de remplissage (6) pour remplir lesdits réservoirs (2) avec un produit pouvant être versé ; ou
- une unité d'obturation (7) pour obturer lesdits récipients (2) contenant ledit produit pouvant être versé ; ou
une combinaison de celles-ci.
